Europäisches Patentamt

European Patent Office

Office européen des brevets

(11) Publication number: **0 249 738**
A1

## EUROPEAN PATENT APPLICATION

(21) Application number: 87106820.1

(22) Date of filing: 11.05.87

(51) Int. Cl.⁴: **G 01 N 22/04**, G 01 N 33/10

(30) Priority: 16.05.86 IT 2046186

(43) Date of publication of application: 23.12.87
Bulletin 87/52

(84) Designated Contracting States: **AT BE CH DE ES FR GB GR IT LI LU NL SE**

(71) Applicant: **OCRIM S.p.A., Via Massarotti, 76, I-26100 Cremona CR (IT)**

(72) Inventor: **Frittoli, Claudio, Via Giordano 121, I-26100 Cremona (IT)**

(74) Representative: **Garrone, Fernando, Internazionale Brevetti s.r.l. Via Brentano 2, I-20121 Milano (IT)**

(54) Device for the continuous measurement of the humidity of food products.

(57) The present invention relates to a device for the continuous measurement of the humidity of food products, particularly suitable for the continuous measurement of the humidity of cereals.

The divice comprises a measurement duct, microwave transmission means arranged at a wall of said duct, and microwave reception means arranged at an opposite wall of said duct; said duct being suspended by means of weighing means with elastic suspensions interposed.

The device allows to perform the continuous analysis of the humidity of food products with high precision and reliability, with no danger of contamination.

# DEVICE FOR THE CONTINUOS MEASUREMENT OF THE HUMIDITY.OF FOOD PRODUCTS

\

The present invention relates to a device for the continuous measurement of the humidity of food products, particularly suitable for the continuous measurement of the humidity of cereals.

The U.S. Patent no. 4.547.725 describes a method and an apparatus for the continuous measurement of the humidity of cereals. A measuring duct is illustrated, in which the cereals flow vertically, its walls being formed by the plates of a capacitor. The measurement of the capacity of the capacitor, suitably processed, provides an indication of the humidity content.

A temperature sensor is provided at the outlet of this measurement duct, and provides data on the temperature of the cereals. A flow sensor capable of providing data on the flow rate or, alternately, a system for weighing the entire measurement duct filled with cereals, provide the data related to the mass of cereals which flows through the measurement duct. The values of these three indexes are then fed to an electronic data processor, which calculates the humidity.

However, it has been observed in practice that the flow of cereals within the measurement duct is not uniform, on the contrary, especially if the surface of the cereals is rather humid, very thick adhesions tend to form proximate to the walls, so that the effective flow is reduced to only the central portion of the duct, and therefore the results of the measurements of the capacitor are biased by the mass of cereals which remains jammed in the adhesions.

The indications of the flow rate measurement device or of the measurement duct weighing device also entail significant imprecisions, due to a series of concurrent and not all fully identifiable reasons, among which the fact that the pressure of the grain upstream of the measurement duct is uncontrollably variable.

The U.S. Patent 4,131,845 describes a humidity measurement device which is based on the known principles according to which (a) a flow of microwaves which passes through a product is attenuated by an amount which depends on the humidity content of the product and (b) a flow of gamma rays which passes through a product is attenuated by an amount which depends on the mass of the product. A container for the product to be analyzed is thus described, provided with means for the transmission and the reception of microwaves and gamma rays which, in combination, allow to determine the percentage humidity content of the product.

However, the use of gamma rays entails remarkable problems of safety and, as a principle, should be discouraged when the product to be analyzed is a food product, which might become contaminated.

An object of the present invention is therefore to provide a device for the continuous control of the humidity of food products which overcomes the above described disadvantages providing precise and reliable measurements even after long periods.

Another object of the invention is to provide a device which does not require the use of radiations which are dangerous or susceptible to contaminate food products.

Not least object of the invention is to allow a particularly simple and economic assembly.

This aim and other objects are achieved by the device according to the invention, which is characterized in that it comprises a measurement duct, microwave transmission means arranged at a wall of said duct, and microwave reception means provided at an opposite wall of said duct; said duct being suspended by means of weighing means with elastic suspensions interposed.

Further characteristics and advantages of the invention will become apparent from the description of a preferred, but not exclusive, embodiment of the device, illustrated only by way of non-limitative example in the accompanying drawing, where:

Fig. 1 is a perspective view of the main body of the device;

Fig. 2 is a lateral cross section view of the device of Fig. 1;

Fig. 3 is a front view of the device of Fig. 1;

Fig 4 is a lateral view of the weighing means.

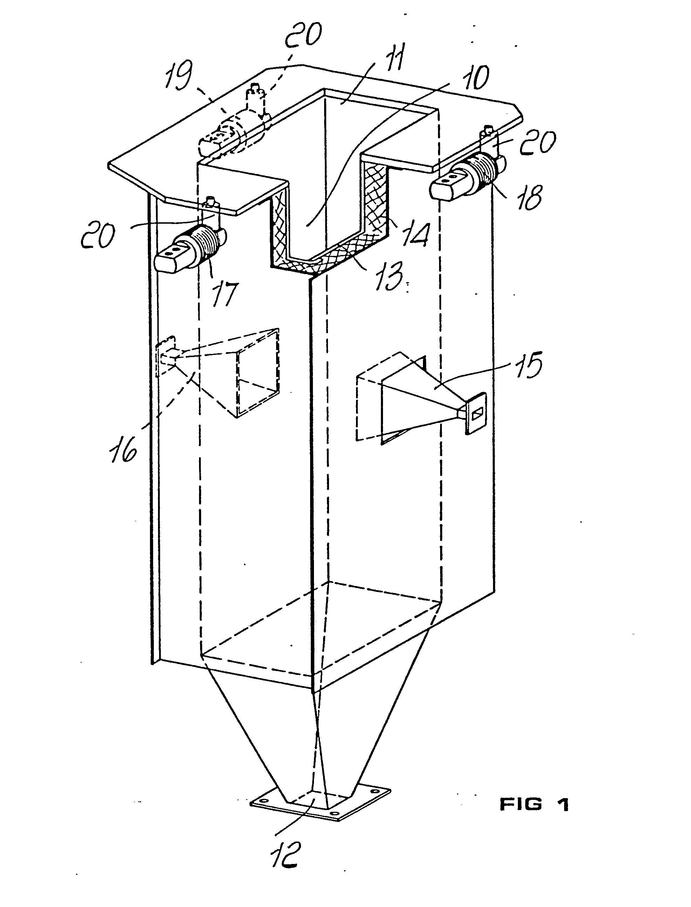

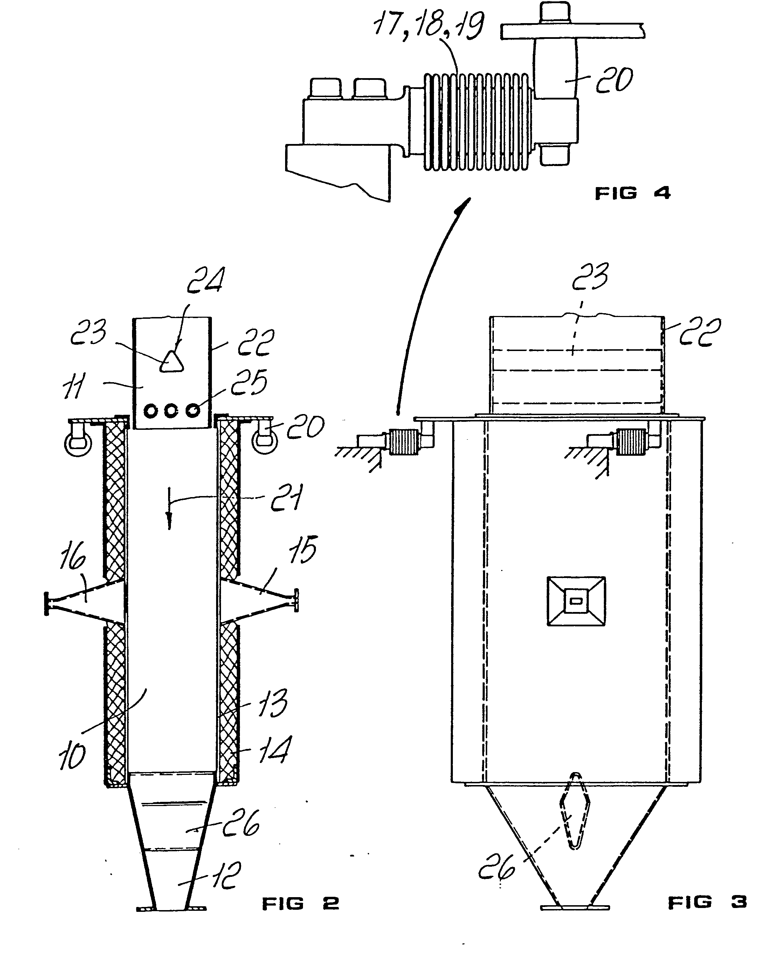

With reference to Figs. 1 to 4, the device comprises a measurement duct 10 inside which the food product to be analyzed is fed continuously. The measurement duct 10 is provided with an inlet 11 and an outlet 12. The inner wall of the duct is provided with a first covering 14 formed by microwave-absorbent material, made, as an example, of a foam marketed by the company Emerson and Cuming Inc. under the trade name Eccosorb. The inner wall of the duct is furthermore provided with a second covering made of microwave-permeable material, placed in direct contact with the food product to be analyzed. The material which forms the second covering 13 is compatible with food product and is preferably made of polycarbonates.

Microwave transmission means 15, arranged at a wall of the duct 10, and microwave reception means 16, arranged at an opposite wall of said duct, are provided. Regarding the configuration of the microwave transmission and reception means 15 and 16, reference is made to the description and to the references of the abovementioned U.S. Patent no. 4,131,845.

The measurement duct 10 is suspended by means of weighing means which are preferably formed by three load cells 17, 18 and 19.

Elastic suspensions are mounted between the load cells 17, 18 and 19 and the measurement duct. Each of said elastic suspensions may be composed, as an example, of an elastomeric body 20. The resilience of said elastic suspensions must be selected so that the frequency of the system composed of the measurement duct full of product and said elastic suspensions is comprised between 0.5 and 4 Hz and is preferably comprised between 1 and 2 Hz.

Solid obstacles are provided and arranged proximate to the inlet 11 of the duct 10. Said obstacles are arranged transversely with respect to the direction 21 of the flow of food products, and are supported by a structure 22 arranged upstream of the measurement duct 10. Said solid obstacles comprise a wedge-shaped body 23, wherein the narrow end 24 is arranged in an opposite direction with respect to the direction 21 of the flow of food products and furthermore comprise a plurality of bars 25 arranged downstream of the wedge-shaped body 26.

Inside the outlet 12 of the duct 10, a solid elongated body 26 is provided, with a longitudinal axis arranged along the direction 21 of the flow of food products. The solid elongated body 26 is preferably provided in the shape of a double wedge or double cone, wherein the upstream wedge or cone is provided with a height which is smaller than that of the downstream wedge or cone.

In practice, it has been observed that the device according to the invention allows the continuous evaluation of the humidity content of food products and, in particular, of cereals. The device furthermore allows to insert upstream a device for wetting the cereals, which is activated automatically when the device according to the invention determines that the humidity content is insufficient.

The device allows the detection of the humidity in a surprisingly precise manner, regardless of the conditions of the surrounding plant, and in particular regardless of the conditions of the flow of cereals upstream or downstream of the device.

## CLAIMS

1. Device for the continuous measurement of the humidity of food products, characterized in that it comprises a measurement duct, microwave transmission means arranged at a wall of said duct, and microwave reception means arranged at an opposite wall of said duct; said duct being suspended by means of weighing means with elastic suspensions interposed.

2. Device according to claim 1, characterized in that said elastic suspensions are provided with a resilience which is selected so that the frequency of the system composed of the measurement duct full of product and of the elastic suspensions is comprised between 0.5 and 4 Hz and is preferably comprised between 1 and 2 Hz.

3. Device according to claim 1, characterized in that said elastic suspensions are formed by elastomeric bodies.

4. Device according to claim 1, characterized in that it comprises solid obstacles arranged proximate to the inlet of said duct, transversely with respect to the direction of the flow of said food products, said solid obstacles being supported by a structure arranged upstream with respect to said measurement duct.

5. Device according to any one of the preceding claims, characterized in that said solid obstacles comprise a wedge-shaped body, wherein the narrow end is arranged in an opposite direction with respect to the direction of the flow of said food products.

6. Device according to any one of the preceding claims, characterized in that said solid obstacles comprise a plurality of bars arranged downstream with respect to said wedge-shaped body.

7. Device according to any one of the preceding claims, characterized in that it comprises, inside the outlet of the measurement duct, a solid elongated body, provided with a longitudinal axis arranged along the direction of the flow of food products.

8. Device according to claim 7, characterized in that said solid elongated body is provided in the shape of a double wedge or double cone, the upstream wedge or cone being provided with a height which is smaller than that of the downstream wedge or cone.

9. Device according to what has been described and illustrated.

FIG 1

17,18,19

20

FIG 4

24

23

22

11

25

20

23

22

21

16

15

13

14

10

26

12

FIG 2

26

FIG 3

0249738

# EUROPEAN SEARCH REPORT

European Patent
Office

Application number

EP 87 10 6820

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 043 137 (GEBRÜDER BÜHLER AG) <br> * abstract; page 1, line 22; page 12, line 36 - page 13, line 28; figures * & US - A - 4 547 725 (Cat. D,A) | 1 | G 01 N 22/04 <br> G 01 N 33/10 |
| A,D | US-A-4 131 845 (I.E. PAKULIS) <br> * abstract; figures; column 1, lines 5-10; column 2, lines 31-37, 56 - column 3, line 23 * | 1 | |
| A | US-A-4 485 284 (I.E. PAKULIS) <br> * whole document * | 1 | |
| A | US-A-4 326 163 (R.L. BROOKE) <br> * whole document * | 1 | TECHNICAL FIELDS SEARCHED (Int. Cl.4) |
| A | DE-A-3 038 725 (W. HEINZ) <br> * claims; page 4, last paragraph; page 5, paragraphs 1, 2; page 5, last paragraph - page 6, paragraph 2; page 7, paragraph 3 - page 8, paragraph 2; figures * | 1 | G 01 N 22/00 <br> G 01 N 33/00 |
| A | EP-A-0 034 459 (SATAKE ENGINEERING CO., LTD.) <br> * page 3, line 10 - page 4, line 18; page 5, line 25 - page 6, line 1; figures * | 1 | |

--- -/-

The present search report has been drawn up for all claims

| Place of search | Date of completion of the search | Examiner |
|---|---|---|
| BERLIN | 25-09-1987 | VINSOME R M |

## European Patent Office

## EUROPEAN SEARCH REPORT

| DOCUMENTS CONSIDERED TO BE RELEVANT | | | Page 2 |
|---|---|---|---|
| Category | Citation of document with indication, where appropriate, of relevant passages | Relevant to claim | CLASSIFICATION OF THE APPLICATION (Int. Cl.4) |
| A | DE-A-3 532 570 (VEB KOMBINAT NAGEMA)<br><br>----- | | |
| | | | TECHNICAL FIELDS SEARCHED (Int. Cl.4) |

The present search report has been drawn up for all claims

| Place of search | Date of completion of the search | Examiner |
|---|---|---|
| BERLIN | 25-09-1987 | VINSOME R M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82